## Europäisches Patentamt
## European Patent Office
## Office européen des brevets

(19)

(11) Veröffentlichungsnummer: **0 033 750**
**B1**

(12) **EUROPÄISCHE PATENTSCHRIFT**

(45) Veröffentlichungstag der Patentschrift:
30.11.83

(51) Int. Cl.³: **A 61 K 6/08**, C 08 F 4/00

(21) Anmeldenummer: **80100639.6**

(22) Anmeldetag: **08.02.80**

(54) **Katalysator zur Herstellung von Dentalkunststoffmassen bzw. von Zahnersatzteilen, dessen Verwendung und Dentalkunststoffmassen.**

(43) Veröffentlichungstag der Anmeldung:
**19.08.81 Patentblatt 81/33**

(45) Bekanntmachung des Hinweises auf die Patenterteilung:
**30.11.83 Patentblatt 83/48**

(84) Benannte Vertragsstaaten:
**AT BE CH DE FR GB IT NL SE**

(56) Entgegenhaltungen:
**CH - A - 473 856
DE - A - 2 701 792
FR - A - 1 385 958
FR - A - 2 094 493
US - A - 3 084 436**

**CHEMICAL ABSTRACTS, Band 65, 1966 Nr. 11, 21 November, Zusammenfassung 16888f Columbus, Ohio, US G. RYOZO et al.: "Studies on the pinacol rearrangement. I. Synthesis of some 2,2'-disubstituted benzo-pinacols and separation of the meso and (+-)-isomers"**

**Die Akte enthält technische Angaben, die nach dem Eingang der Anmeldung eingereicht wurden und die nicht in dieser Patentschrift enthalten sind.**

(73) Patentinhaber: **Etablissement Dentaire IVOCLAR, FL-9494 Schaan (LI)**

(72) Erfinder: **Michl, Rudy, Dr., Troexlegass 17, FL-9494 Schaan (LI)**
Erfinder: **Willi, Hanspeter, Eggasweg 6, FL-9490 Vaduz (LI)**

(74) Vertreter: **Splanemann, Rainer, Dipl.-Ing., Patentanwälte Dipl.-Ing. R. Splanemann, Dr. B. Reitzner Tal 13, D-8000 München 2 (DE)**

**0 033 750**

## Katalysator zur Herstellung von Dentalkunststoffmassen bzw. von Zahnersatzteilen, dessen Verwendung und Dentalkunststoffmassen

Die Erfindung betrifft Dentalkunststoffmassen zur Herstellung von Zahnersatzteilen, enthaltend eine oder mehrere Methacrylverbindungen sowie daraus hergestellte Zahnersatzteile.

Es ist bekannt, zur Herstellung von Dentalkunststoffmassen bzw. von Zahnersatzteilen Katalysatoren bzw. Initiatoren auf der Basis von organischen Peroxiden bzw. Benzpinakolen zu verwenden. Dabei kommen z. B. Vinylverbindungen und deren Polymerisate, insbesondere monomere Acrylate und Methacrylate allein oder in Mischung mit Polymeren zum Einsatz. Beispiele für geeignete Katalysatoren sind Benzoylperoxid (BPO), Lauroylperoxid (LPO), unsubstituiertes Benzpinakol und 4,4'-Dimethylbenzpinakol. Bei Verwendung von Benzoylperoxid bilden sich jedoch sehr häufig viele kleine Blasen, die den polymerisierten Körper oft gleichmäßig durchziehen. Diese Blasen führen einmal zu einer Herabsetzung der Festigkeit des polymerisierten Körpers, zum anderen zu einer Herabsetzung der Transparenz. Die Transparenz der Polymerisate ist besonders wichtig bei künstlichen Zähnen, insbesondere bei Schneidezähnen, wo schon eine geringe Anzahl von feinen Bläschen zu ästhetisch unbrauchbaren Produkten führt. Die Anwesenheit von kleinen Bläschen ist ferner auch in Zahnprothesen, sowie in allen anderen polymerisierten Zahnersatzteilen unerwünscht. Außerdem haben die Peroxide generell die Eigenschaft, sich bei längerer Lagerung zu zersetzen bzw. im Endprodukt dann eine unerwünschte Verfärbung hervorzurufen.

Ferner sind aus der DE-A-2 701 792 in der Wärme aktivierbare, äthylenisch ungesättigte Monomere (insbesondere ungesättigte Polyester) bekannt, die als Katalysator ein Pinakol (z. B. Benzpinakol) enthalten. Diese Monomere eignen sich jedoch nicht zur Herstellung von Dentalkunststoffmassen.

Aus der CH-A-473 856 sind bei Raumtemperatur lagerfähige, durch Erwärmung härtbare Polyester-Formmassen bekannt, die als Katalysatoren verschiedenartige Benzpinakole enthalten können. Eine Bevorzugung von 2,2'-Dialkylbenzpinakolen ist nicht erkennbar; außerdem eignen sich die Polyester nicht zur Herstellung von Dentalkunststoffmassen.

Aus der FR-A-1 385 958 ist ein Verfahren zur Herstellung von stabilen Vorpolymeren aus verschiedenartigen, einfach oder mehrfach ungesättigten Monomeren unter Verwendung von verschiedenartigen Benzpinakolen als Katalysatoren bekannt. Eine Bevorzugung von 2,2'-Dialkylbenzpinakolen oder bestimmten Monomeren ist nicht erkennbar. Es finden sich auch keine Hinweise auf die Eignung der Monomeren bzw. der Vorpolymeren zur Herstellung von Dentalkunststoffmassen.

Generell versteht man unter Dentalkunststoffmassen alle polymerisierbaren und polymerisierten Produkte ohne eine bestimmte Form; unter Zahnersatzteilen versteht man die entsprechenden geformten Produkte, die entweder aus den polymerisierbaren oder den polymerisierten Dentalkunststoffmassen unter Formgebung hergestellt werden, wie zum Beispiel künstliche Zähne, Kronen und Brücken sowie Prothesen und dergleichen.

Werden statt der Peroxide die Benzpinakole als Katalysatoren bei der Herstellung von Dentalkunststoffmassen bzw. von Zahnersatzteilen verwendet, so ist die Gefahr der Bildung kleinerer Blasen zwar nicht so ausgeprägt. Es bilden sich jedoch häufig bei der Polymerisation in unkontrollierbarer Weise größere Blasen in geringer Anzahl.

Der Erfindung liegt die Aufgabe zugrunde, bei der Herstellung von Dentalkunststoffmassen bzw. von Zahnersatzteilen die Polymerisation von Methacrylverbindungen so zu steuern, daß keine unerwünschte Blasenbildung im Polymerisat erfolgt. Außerdem sollen unerwünschte Verfärbungen im Endprodukt weitgehend vermieden werden.

Gegenstand der Erfindung sind Dentalkunststoffmassen zur Herstellung von Zahnersatzteilen, enthaltend:

(a₁) eine oder mehrere polymerisierbare Methacrylverbindungen, gegebenenfalls im Gemisch mit Polymerisationsinhibitoren; oder

(a₂) aus (a₁) durch Polymerisation hergestellte Dentalkunststoffmassen; oder Gemische aus (a₁) und (a₂) und

(b) einen Katalysator, bestehend aus oder enthaltend mindestens ein(em) 2,2'-Dialkylbenzpinakol, worin die Alkylgruppe 1 bis 6 Kohlenstoffatome enthält.

Die erfindungsgemäßen Massen sind insbesondere dadurch gekennzeichnet, daß der Katalysator aus 2,2'-Dimethylbenzpinakol besteht bzw. dieses enthält.

Das 2,2'-Dimethylbenzpinakol ist an sich bekannt; seine Herstellung ist beispielsweise in »Journal of American Chemical Society«, Band 52, Seiten 3596—3603 (1930) beschrieben. Diese Literaturstelle beschreibt insbesondere den Einfluß von Substituenten auf die Umlagerung von Benzpinakol zu Benzpinakon. Hinweise auf die Eignung als Katalysatoren zur Polymerisation von monomeren Methacrylaten finden sich jedoch in dieser Literaturstelle nicht.

Die erfindungsgemäße Wirkung der 2,2'-Dialkylbenzpinakole, insbesondere des 2,2'-Dimethylbenzpinakols, ist auch insofern überraschend, als das strukturell ähnliche 4,4'-Dimethylbenzpinakol als Katalysator für den vorliegenden Zweck unbrauchbar ist.

2

Der erfindungsgemäß verwendete Katalysator kann neben dem 2,2'-Dialkylbenzpinakol noch eine oder mehrere der nachstehend angegebenen Zusatzkomponenten enthalten:

    2,2'-Dichlorbenzpinakol
       Benzpinakol
    2,2'-Dibrombenzpinakol
    2,2'-Dicyanobenzpinakol.

Die beiden zuerst genannten Verbindungen werden bevorzugt.

Der besondere Vorteil bei der Verwendung von Katalysatorgemischen liegt in der besseren Steuerbarkeit des Polymerisationsvorganges. Außerdem hängt die Verwendung von Gemischen weitgehend davon ab, welchen Dentalkunststoff man herstellen möchte. Im allgemeinen kann der Anteil der Zusatzkomponente bis zu 80 Gew.-%, vorzugsweise 10 bis 60 Gew.-%, bezogen auf den gesamten Katalysator, betragen.

Setzt man den Katalysator für ein Prothesenmaterial ein, so erhält man gute Ergebnisse mit einem Gemisch aus 20 Gew.-% 2,2'-Dimethylbenzpinakol und 80 Gew.-% Benzpinakol. Vorzugsweise wird jedoch der Gehalt an 2,2'-Dimethylbenzpinakol erhöht, wobei man mit 40 Gew.-% sehr gute Ergebnisse erzielt.

Setzt man den erfindungsgemäß verwendeten Katalysatortyp für ein Kronen- und Brückenmaterial ein, so kann man brauchbare Ergebnisse mit einer Mischung aus 70 Gew.-% 2,2'-Dimethylbenzpinakol und 30 Gew.-% 2,2'-Dichlorbenzpinakol erzielen. Bessere Ergebnisse werden erzielt, wenn man den Gehalt an 2,2'-Dimethylbenzpinakol erhöht, vorzugsweise auf 80 Bis 90 Gew.-%.

Als Beispiele für die polymerisierbaren Methacrylate kommen Verbindungen in Frage, die z. B. in den DE-A-2 818 068, 2 419 887, 2 126 419 und 2 728 764 genannt sind. Große Bedeutung haben das Glycidylmethacrylatderivat von Bisphenol A(Bis-GMA) sowie die bekannten Modifikationen dieses Harzes. Die polymerisierbaren Methacrylate können allein oder als Gemische eingesetzt werden. Stellvertretend für die Vielzahl dieser bekannten Verbindungen seien genannt:

### (a) Monomethacrylate

— Methylmethacrylat
— Äthylmethacrylat
— Isopropylmethacrylat
— n-Hexylmethacrylat
— Hydroxyäthylmethacrylat

### (b) Mehrfunktionelle Methacrylate

— Äthylenglykoldimethacrylat
— Butandiol 1,4 Dimethacrylat
— Triäthylenglykoldimethacrylat
— Dodecandiol 1,12 Dimethacrylat
— Decandiol 1,10 Dimethacrylat
— 2,2 bis-[-p($\gamma$-Methacryloxy-$\beta$-hydroxypropoxy)phenyl]propan
— Diaddukt aus Hydroxyäthylmethacrylat und Trimethylhexamethylendiisocyanat
— Diaddukt aus Hydroxyäthylmethacrylat und Isophorondiisocyanat
— Trimethylolpropantrimethacrylat
— Pentaerythrittrimethacrylat
— Pentaerythrittetramethacrylat
— 2,2-Bis-/p($\beta$-hydroxy-äthoxy)phenyl/propan-dimethacrylat.

Der Katalysator ist in den erfindungsgemäßen Massen zweckmäßig in Mengen von 0,05 bis 5 Gew.-%, vorzugsweise von 0,2 bis 1,5 Gew.-%, bezogen auf die polymerisierbare(n) Methacrylverbindung(en) vorhanden.

Die polymerisierbaren Dentalkunststoffmassen sind im allgemeinen dadurch erhältlich, daß man die vorstehend definierte polymerisierbare Masse, welche die Komponente ($a_1$) oder ein Gemisch der Komponenten ($a_1$) und ($a_2$) enthält, bei Temperaturen im Bereich von 70 bis 180° C, vorzugsweise von 75 bis 120°C, polymerisiert. Die Polymerisationstemperatur hängt hauptsächlich ab von dem zu polymerisierenden Monomer und der Katalysatorkonzentration sowie von der Schnelligkeit, mit der ein Material polymerisiert werden muß. Bei der Polymerisation von Methylmethacrylat für Zahnprothesen wird beispielsweise in einem Temperaturbereich von 90 bis 100° C gearbeitet, wenn die Katalysatorkonzentration 1 Gew.-%, bezogen auf das Methylmethacrylat, beträgt.

Es wurde ferner festgestellt, daß die Polymerisationstemperatur nach dem Anspringen der

3

Polymerisationsreaktion herabgesetzt werden kann, wobei die Reaktion verlangsamt oder sogar unterbrochen werden kann. Auf diese Weise kann die Verarbeitungszeit der Zahnersatzteile verlängert werden bzw. es können Fehler bei der Formgebung der Zahnersatzteile korrigiert werden. Dies ist ein großer Vorteil gegenüber den Peroxid-Katalysatoren, bei denen die Polymerisation, wenn sie einmal begonnen hat, nicht mehr unterbrochen werden kann.

Gegenstand der Erfindung sind ferner Zahnersatzteile in Form von künstlichen Zähnen, Kronen, Brücken und Zahnprothesen, die aus den vorstehend definierten polymerisierbaren bzw. polymerisierten Massen hergestellt sind.

Die Herstellung der 2,2'-Dialkylbenzpinakole erfolgt in an sich bekannter Weise. Ein bevorzugtes Verfahren zur Herstellung des 2,2'-Dimethylbenzpinakols besteht darin, daß 2-Methylbenzophenon mit Zinkstaub und Essigsäure reduziert wird. Dabei wird 2-Methylbenzophenon 16 Stunden in einem Gemisch aus Isopropanol, Wasser. Essigsäure und Zinkstaub unter Luftausschluß gerührt. Die Isolierung des 2,2'-Dimethylbenzpinakols erfolgt in bekannter Weise durch Extraktion mit Methylenchlorid. Man erhält ein festes Produkt mit einem Schmelzpunkt von 160°C.

Die Katalysatoren können also den polymerisierbaren Methacrylverbindungen zugesetzt werden. Die so erhaltenen Gemische sind einige Zeit stabil, insbesondere wenn man gewisse Vorsichtsmaßnahmen einhält. Beispielsweise kann man die Stabilität der Gemische dadurch erhöhen, daß man sie bei tiefen Temperaturen und/oder unter Lichtausschluß lagert. Vorzugsweise setzt man Polymerisationsinhibitoren in geringen Mengen zu. Bei der Anwendung wird die Polymerisation dann im allgemeinen durch Erhitzen oder Bestrahlen des Gemischs bzw. durch Zusatz von Aktivatoren, die die Wirkung der Inhibitoren aufheben, in Gang gesetzt.

Die erfindungsgemäßen Massen können aber auch als Gemische der aus den polymerisierbaren Methacrylverbindungen durch Polymerisation hergestellten Dentalkunststoffmassen und den Katalysatoren in den Handel gebracht werden. Das Gemisch aus Polymer und Katalysator ist über einen langen Zeitraum stabil. Der Anwender kann dann unmittelbar vor der Verarbeitung diesem Gemisch polymerisierbare Methacrylverbindungen zusetzen, wobei die monomeren Verbindungen zunächst als Lösungsmittel für die Polymeren dienen und anschließend mit Hilfe des Katalysators polymerisiert werden. In den auspolymerisierten Zahnersatzteilen können dann zwei verschiedene Polymere vorliegen, bzw. die Monomeren können auf das ursprünglich vorhandene Polymer aufgepfropft werden.

Das Gemisch aus Ausgangspolymer und Katalysator sowie das später zuzusetzende Monomer können auch in getrennten Kammern von Mehrkomponentenbehältern enthalten sein. Unmittelbar vor der Anwendung werden die Trennwände zwischen den einzelnen Kammern entfernt, und die nun vereinigten Substanzen werden gründlich miteinander vermischt, was beispielsweise mit Hilfe von hochtourigen Schwingmischvorrichtungen geschehen kann.

Der Katalysator kann auch getrennt in einer dritten Kammer des mehrkammerigen Behälters untergebracht sein.

Die erfindungsgemäßen Massen können noch andere Bestandteile, beispielsweise Farbstoffe und feinteilige anorganische Füllstoffe, enthalten.

Die Erfindung ist durch die nachstehenden Beispiele erläutert.

## Beispiel 1

In diesem Beispiel wird die Wirkung des 2,2'-Dimethylbenzpinakols als Polymerisationskatalysator mit der von Benzoylperoxid (BPO), unsubstituiertem Benzpinakol und 4,4'-Dimethylbenzpinakol verglichen.

Zur Durchführung der Vergleichsversuche wurde aus 30 g peroxidfreiem Polymethylmethacrylat, das in Form eines feinteiligen Perlpolymersats vorlag, und 15 g eines flüssigen Gemisches aus 90 Gew.-% Methylmethacrylat und 10 Gew.-% Äthylenglykoldimethacrylat ein pastenförmiges Gemisch hergestellt. Diesem Gemisch wurde jeweils ein Katalysator bzw. ein Katalysatorgemisch zugesetzt, wie es aus der nachfolgenden Tabelle ersichtlich ist. Die Katalysatormenge bezieht sich auf das flüssige Monomer und ist in Gew.-% angegeben. Anschließend wurde das mit dem Katalysator versetzte Gemisch in einer Küvette zu einem Sechskantriegel mit einer Kantenlänge von 10,4 mm und einer Länge von etwa 70 mm polymerisiert. Die Polymerisation wurde im Wasserbad bei einer Temperatur von 100°C während 1 Std. durchgeführt. Danach wurde die Küvette im kalten Wasser abgekühlt, die Sechskantriegel ausgebettet und über die Länge in 4 gleiche Teile zersägt.

Die Ergebnisse sind in der nachstehenden Tabelle angegeben.

| Katalysator | Polymeri-sationszeit, Min. | Gew.-% Katalysator bzw. Kat.-Gemisch | Ergebnisse |
|---|---|---|---|
| 1. Benzoylperoxid | 60 | 1 | Durch den ganzen Prüfkörper Blasenbalken und feine Blasen |
| 2. Benzpinakol | 60 | 1 | Große, blätterartige Blasen durch den Prüfkörper |
| 3. 4,4'-Dimethylbenzpinakol | 60 | 1 | Große Blasen in der Mitte des Prüfkörpers |
| 4. 2,2'-Dimethylbenzpinakol | 60 | 1 | Fast keine Blasen |
| 5. 2,2'-Dimethylbenzpinakol und Benzpinakol | 60 | 0,4 ⎱ 0,6 ⎰ | Keine Blasen |
| 6. 2,2'-Dimethylbenzpinakol und 2,2'-Dichlorbenzpinakol | 60 | 0,8 ⎱ 0,2 ⎰ | Sehr wenig Blasen |

Die Versuche 4—6 sind erfindungsgemäß.

## Beispiel 2

In ein Reagenzglas wurde in flüssiges Methacrylatgemisch, bestehend aus 90 Gew.-% Methylmethacrylat und 10 Gew.-% Äthylenglykoldimethacrylat eingefüllt. Die polymere Komponente wurde also weggelassen. Dem flüssigen Gemisch (etwa 20 cm$^3$) wurden jeweils folgende Katalysatoren zugesetzt:

1.  1 Gew.-%      2,2'-Dimethylbenzpinakol
2.  0,3 Gew.-%      2,2'-Dimethylbenzpinakol
3.  0,5 Gew.-%      2,2'-Dimethylbenzpinakol und 2 Gew.-% Benzpinakol

Die mit den Katalysatoren versetzten Gemische wurden bei etwa 70°C 24 Stunden in einem Ofen stehengelassen. Die polymerisierten Körper im Fall 1 waren blasenfrei, im Fall 2 waren sehr wenig Blasen vorhanden. Im Fall 3 war der Prüfkörper ebenfalls blasenfrei. Das Ergebnis von Fall 3 ist insofern überraschend, als der Gehalt an 2,2'-Dimethylbenzpinakol ziemlich niedrig liegt und der Zusatz von Benzpinakol, das nach Beispiel 1 allein ein schlechtes Ergebnis liefert, die Wirkung des 2,2'-Dimethylbenzpinakols nicht verschlechtert. Andererseits polymerisiert das Gemisch 3 schneller aus als das Gemisch 1.

## Beispiel 3

Die Reaktivität des 2,2'-Dimethylbenzpinakols wurde im Vergleich zu Benzoylperoxid, Benzpinakol und 4,4'-Dimethylbenzpinakol bestimmt. Dazu werden 4 Lösungen hergestellt, bestehend aus einer Mischung aus 90 Gew.-% Methylmethacrylat, stabilisiert mit 50 ppm Hydrochinon und 10 Gew.-% Äthylenglykoldimethacrylat mit jeweils 0,025 Mol Katalysator pro kg Gemisch. Mit diesen Lösungen werden DTA-Diagramme aufgenommen.
Daten des Gerätes:

Perkin-Elmer DSC-2
Empfindlichkeit 5 mcal/s
Aufheizgeschwindigkeit: 320 K/min (293 − 372 K)
Schreibervorschub: 1 cm/min

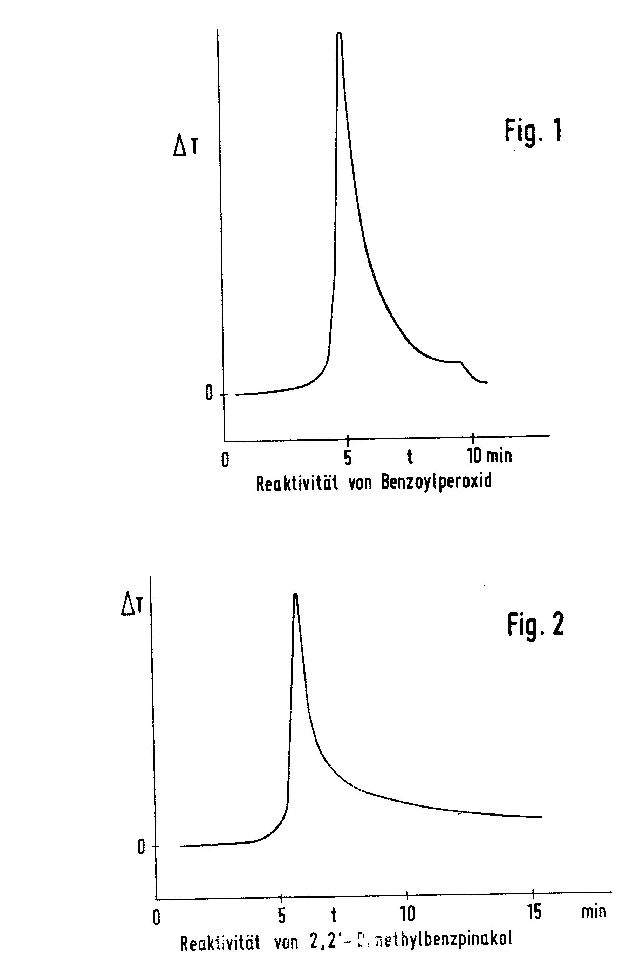

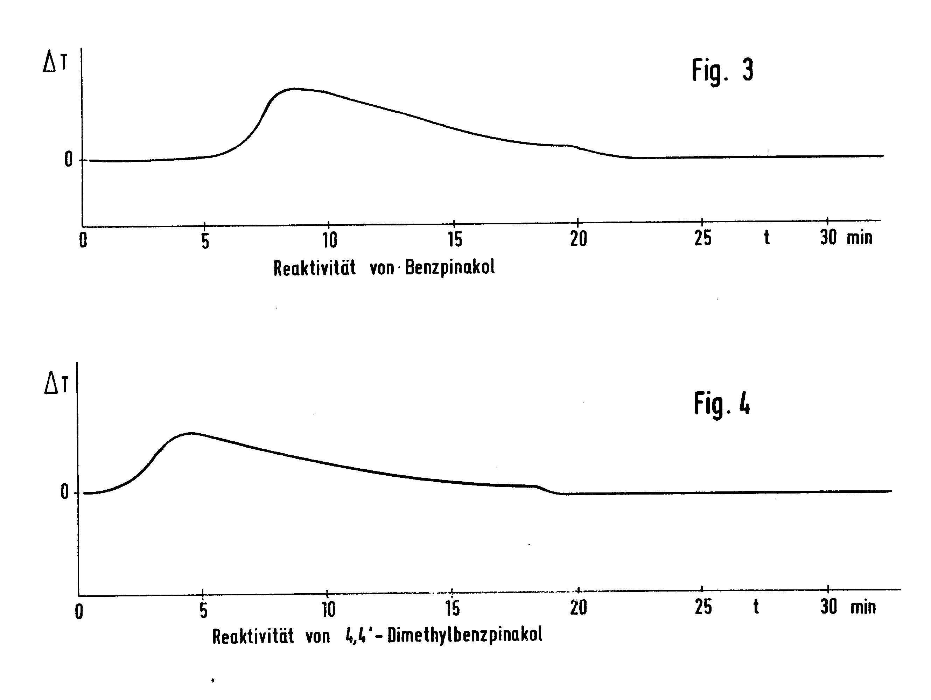

Die erhaltenen Kurven (Fig. 1−4) zeigen die Wärmeabgabe (ausgedrückt als Temperaturerhöhung $\Delta T$ in willkürlichen Einheiten) als Funktion der Zeit (t in min). Bei Benzpinakol (Fig. 3) und

**0 033 750**

4,4'-Dimethylbenzpinakol (Fig. 4) läuft die Polymerisation über einen längeren Zeitraum, während sie bei Benzoylperoxid (Fig. 1) schlagartig abläuft. Trotz der relativ flachen Polymerisationskurven von Fig. 3 und 4 zeigten die Proben eine Blasenbildung, vergleichbar mit den Proben 2 und 3 von Beispiel 1.

Das 2,2'-Dimethylbenzpinakol (Fig. 2) bewirkt, verglichen mit Benzoylperoxid, einen gemäßigten Polymerisationsverlauf, verbunden mit einer relativ kurzen Polymerisationsdauer. Eine Blasenbildung konnte nicht beobachtet werden.

**Patentansprüche**

1. Dentalkunststoffmassen zur Herstellung von Zahnersatzteilen, enthaltend

($a_1$) eine oder mehrere polymerisierbare Methacrylverbindungen, gegebenenfalls im Gemisch mit Polymerisationsinhibitoren; oder

($a_2$) aus ($a_1$) durch Polymerisation hergestellte Dentalkunststoffmassen; oder Gemische aus ($a_1$) und ($a_2$) und

(b) einen Katalysator, bestehend aus oder enthaltend mindestens ein(em) 2,2'-Dialkylbenzpinakol, worin die Alkylgruppe 1 bis 6 Kohlenstoffatome enthält.

2. Massen nach Anspruch 1, dadurch gekennzeichnet, daß der Katalysator aus 2,2'-Dimethylbenzpinakol besteht bzw. dieses enthält.

3. Massen nach Anspruch 1 oder 2, dadurch gekennzeichnet, daß der Katalysator als Zusatzkomponente 2,2'-Dichlorbenzpinakol und/oder Benzpinakol enthält.

4. Massen nach Anspruch 3, dadurch gekennzeichnet, daß der Anteil der Zusatzkomponente bis zu 80 Gew.-%, bezogen auf den gesamten Katalysator, beträgt.

5. Massen nach Anspruch 4, dadurch gekennzeichnet, daß der Anteil der Zusatzkomponente 10 bis 60 Gew.-%, bezogen auf den gesamten Katalysator, beträgt.

6. Massen nach einem der Ansprüche 1 bis 5, dadurch gekennzeichnet, daß der Katalysator in Mengen von 0,05 bis 5 Gew.-%, bezogen auf die polymerisierbare(n) Methacrylverbindung(en) vorhanden ist.

7. Massen nach Anspruch 6, dadurch gekennzeichnet, daß der Katalysator in Mengen von 0,2 bis 1,5 Gew.-%, bezogen auf die polymerisierbare(n) Methacrylverbindung(en) vorhanden ist.

8. Polymerisierte Dentalkunststoffmassen zur Herstellung von Zahnersatzteilen, dadurch erhältlich, daß man eine Masse nach einem der Ansprüche 1 bis 7, welche die Komponente ($a_1$) oder ein Gemisch der Komponenten ($a_1$) und ($a_2$) enthält, bei Temperaturen im Bereich von 70 bis 180°C polymerisiert.

9. Dentalkunststoffmassen nach Anspruch 8, dadurch gekennzeichnet, daß man die Polymerisation bei 75 bis 120°C durchführt.

10. Polymerisierte Masse nach Anspruch 8, dadurch erhältlich, daß die Polymerisationstemperatur nach dem Anspringen der Polymerisationsreaktion herabgesetzt wird.

11. Zahnersatzteile in Form von künstlichen Zähnen, Kronen, Brücken und Zahnprothesen, hergestellt aus den Massen nach einem der Ansprüche 1 bis 10.

**Claims**

1. Dental synthetic compositions for the production of dental prosthetic parts, containing

($a_1$) one or more polymerisable methacrylic compounds, optionally in mixture with polymerisation inhibitors; or

($a_2$) dental synthetic compositions produced from ($a_1$) by polymerisation; or mixtures of ($a_1$) and ($a_2$) and

(b) a catalyst consisting of or containing at least one 2,2'-dialkylbenzopinacol, wherein the alkyl group contains 1 to 6 carbon atoms.

2. Compositions according to Claim 1, characterised in that the catalyst consists of 2,2'-dimethylbenzopinacol or contains this latter.

3. Compositions according to Claim 1 or 2, characterised in that the catalyst contains as additional component 2,2'-dichlorobenzopinacol and/or benzopinacol.

4. Compositions according to Claim 3, characterised in that the proportion of the additional component amounts to as much as 80% by weight, in relation to the total catalyst.

5. Compositions according to Claim 4, characterised in that the proportion of additional component amounts to 10 to 60% by weight, in relation to the total catalyst.

6. Compositions according to one of Claims 1 to 5, characterised in that the catalyst is present in quantities of from 0.05 to 5% by weight, in relation to the polymerisable methyacrylic compound(s).

6

7. Compositions according to Claim 6, characterised in that the catalyst is present in quantities of from 0.2 to 1.5% by weight, in relation to the polymerisable methacrylic compound(s).

8. Polymerised dental synthetic compositions for the production of dental prosthetic parts, obtainable by polymerising at temperatures within a range of 70 to 180°C a composition according to one of Claims 1 to 7, which composition contains the component $(a_1)$ or a mixture of the components $(a_1)$ and $(a_2)$.

9. Dental synthetic compositions according to Claim 8, characterised in that the polymerisation is carried out at 75 to 120°C.

10. Polymerised composition according to Claim 8, obtainable by reducing the polymerisation temperature after the starting of the polymerisation reaction.

11. Dental prosthetic parts in the form of artificial teeth, crowns, bridges and dental prostheses produced from the compositions according to one of Claims 1 to 10.

**Revendications**

1. Matières synthétiques dentaires destinées à la réalisation d'éléments dentaires de remplacement, comprenant:

$(a_1)$ un ou plusieurs composés méthacryliques polymérisables, éventuellement en mélange avec des inhibiteurs de polymérisation; ou

$(a_2)$ des matières synthétiques dentaires réalisées à partir de $(a_1)$ par polymérisation; ou des mélanges de $(a_1)$ et $(a_2)$ et

(b) un catalyseur, constitué par ou contenant au moins un 2,2'-dialkylbenzpinacol, où le groupe alcoyle contient de 1 à 6 atomes de carbone.

2. Matières selon la revendication 1, caractérisées en ce que le catalyseur est constitué par du 2,2'-diméthylbenzpinacol ou contient ce dernier.

3. Matières selon la revendication 1, caractérisées en ce que le catalyseur contient en tant que composant additionnel du 2,2'-dichlorbenzpinacol et/ou du benzpinacol.

4. Matières selon la revendication 3, caractérisées en ce que la fraction de composant additionnel représente jusqu'à 80% en poids, par rapport au catalyseur total.

5. Matières selon la revendication 4, caractérisées en ce que la fraction de composant additionnel représente de 10 à 60% en poids, par rapport au catalyseur total.

6. Matières selon l'une quelconque des revendications 1 à 5, caractérisées en ce que le catalyseur se présente selon des quantités comprises entre 0,05 et 5% en poids, par rapport aux composés méthacryliques polymérisables.

7. Matières selon la revendication 6, caractérisées en ce que le catalyseur est présent selon des quantités comprises entre 0,2 et 1,5% en poids, par rapport au ou aux composés méthacryliques polymérisables.

8. Matières synthétiques dentaires polymérisées destinées à la fabrication d'éléments dentaires de remplacement, pouvant être obtenues en polymérisant à des températures comprises dans la plage de 70 à 180°C une matière selon l'une quelconque des revendications 1 à 7, qui contient le composant $(a_1)$ ou un mélange des composants $(a_1)$ et $(a_2)$.

9. Mathières synthétiques dentaires selon la revendication 8, caractérisées en ce que l'on fait se dérouler la polymérisation entre 75 et 120°C.

10. Matière polymérisée selon la revendication 8, caractérisée en ce qu'on l'obtient en abaissant la température de la polymérisation après le démarrage de la réaction de polymérisation.

11. Eléménts dentaires de remplacement se présentant sous la forme de dents, de couronnes, de bridges et de prothèses dentaires synthétiques, fabriquées avec les matières selon l'une quelconque des revendications 1 à 10.

Fig. 1

Reaktivität von Benzoylperoxid

Fig. 2

Reaktivität von 2,2'-Dimethylbenzpinakol

Fig. 3

Reaktivität von Benzpinakol

Fig. 4

Reaktivität von 4,4'-Dimethylbenzpinakol

0 033 750